# EUROPÄISCHE PATENTSCHRIFT

(11) **EP 2 226 039 B1**
(45) Veröffentlichungstag und Bekanntmachung des Hinweises auf die Patenterteilung: **25.11.2015**
(21) Anmeldenummer: 10000688.1
(22) Anmeldetag: 25.01.2010
(51) Int. Cl.: A61F 2/44, A61B 17/70

(54) **Implantat für die Brust- und Lendenwirbelsäule**
Implant for the thoracic and lumbar spine
Implant pour colonne dorsale et lombaire

(30) Priorität: 06.03.2009 DE 102009011563
(43) Veröffentlichungstag der Anmeldung: 08.09.2010
(73) Patentinhaber: Böhm, Heinrich, Dr., 99425 Weimar (DE)
(72) Erfinder: Burger, Andreas, 78532 Tuttlingen (DE); Wenzler, Klaus, 78665 Frittlingen (DE); Böhm, Heinrich, Dr., 99425 Weimar (DE)
(74) Vertreter: Mammel, Ulrike

(56) Entgegenhaltungen:
- WO-A1-98/34568
- WO-A1-2007/087477
- WO-A2-01/56497
- WO-A2-02/062272
- WO-A2-2004/037067
- FR-A1- 2 771 282
- US-A- 6 159 211
- US-A1- 2002 052 656
- US-A1- 2002 068 976
- US-A1- 2007 270 954
- US-A1- 2007 270 968
- US-B1- 6 190 414
- US-B1- 6 491 724

## Beschreibung

Die vorliegende Erfindung betrifft ein Implantat für die Brust- und Lendenwirbelsäule.

Operative Versteifungen von erkrankten Bewegungssegmenten der Wirbelsäule sind seit vielen Jahrzehnten als Standardverfahren an Lenden-, Brust- und Halswirbelsäule etabliert.

Dabei wird unter Opferung der Bewegungsfunktion nach der Ausheilung die Belastbarkeit und weitestgehend auch Schmerzfreiheit wiederhergestellt. Wegen der exzentrischen Lage des Tragegerüsts im Körper kann es sowohl nach operativen Versteifungen als auch bei manchen Erkrankungen (z.B.: Morbus Bechterew) zu zunehmenden Fehlstellungen kommen: Der Wirkung der Schwerkraft folgend verbiegt sich der mechanisch überlastete Wirbelsäulenabschnitt im Sinne einer Kyphose.

Zur Korrektur ist - ähnlich wie bei einem Röhrenknochen - eine Keilosteotomie erforderlich: Dabei ist es essentiell den Drehpunkt der Aufrichtung in den Bereich des Rückenmarks zu verlagern, damit Letzteres den geringstmöglichen Längenänderungen ausgesetzt wird.

Es wird also in den dorsalen Strukturen, den Wirbelbögen, ein Keil entnommen, so dass nach einer Durchtrennung der vor dem Rückenmark liegenden Strukturen durch Schluss des Keildefekts eine dosierte Korrektur der Gesamtsäule möglich ist.

Im Ergebnis klafft im Bereich der vorderen Säule ein Defekt in Form eines gleichschenkeligen Dreiecks bzw. dreidimensional gesehen, in Form eines Orangerischnitzes.

Bei jedem keilförmigen Implantat, das man in einen solchen Defekt einbringt, besteht die Gefahr, dass es sich disloziert. Deshalb muss versucht werden, das Implantat fest mit den angrenzenden Wirbelkörpern zu verschrauben. Aus operationstechnischen Gründen - der Körper kann an den infragekommenden Abschnitten nicht von beiden Seiten geöffnet oder die Wirbelsäule von vorne ausreichend freigelegt werden - ist dies in der Regel nur von einer Seite möglich, was mechanisch ungünstig ist.

Aus der US 7 217 291 B2 ist ein Wirbelsäulenimplantat mit einem Implantatkörper bekannt, das an der Ober- und Unterseite des Implantatkörpers Zähne aufweist, die der Verankerung in Zugrichtung dienen und ein Stellelement, das zum Aufspreizen des Implantatkörpers dient.

Die US 2008/029575 A1 lehrt ein Implantat, das mittels Knochenschrauben verankert wird und nicht spreizbar ist.

Aus der WO 02/062272 und der WO 01/56497 ist ein Implantatkörper mit zwei Schenkeln bekannt, zwischen denen ein Spreizelement vorgesehen ist, wobei der Implantatkörper dazu dient, zwischen zwei Wirbelkörpern eingebracht zu werden. Zur Verankerung können Schrauben, die sich von der Innenseite des Implantatschenkels durch den Schenkel in Richtung der benachbarten Wirbelkörper erstrecken, eingesetzt werden.

Die US 2002/0052756 A1 lehrt ebenfalls ein zweischenkliges Implantat, das ein Spreizelement und Verankerungselemente, die sich von der Innenseite des Implantatschenkels durch den Schenkel in Richtung der benachbarten Wirbelkörper erstrecken, aufweist.

Die US 2007/0270968 lehrt ein Implantat mit zwei Schenkeln und einem keilförmigen Spreizelement.

Das Implantat aus der WO 98/34568 umfasst zwei Schenkel (42), zwischen denen ein Spreizelement (54) vorgesehen ist und ein Element, das zum Aufspreizen der Schenkel (42) dient. Die Verankerung erfolgt bei diesem Implantat durch aufgeraute Wandelemente.

Die US 2007/0270954 A1 lehrt ein zylindrisches Wirbelsäuleimplantat mit zwei Schenkeln und einem Spreizelement, das mittels eines Handwerkzeugs aufgespreizt wird.

Die Aufgabe der Erfindung besteht darin, ein Implantat bereit zu stellen, bei dem die Gefahr einer Verschiebung vermieden wird.

Diese Aufgabe wird durch die Merkmale des Anspruchs 1 gelöst.

Erfindungsgemäß umfasst das Wirbelsäulenimplantat neben dem keilförmigen Implantatkörper, der zwischen zwei Wirbelkörpern einbringbar ist, wobei der Implantatkörper zwei Schenkel aufweist, zwischen denen ein Spreizelement vorgesehen ist" wenigstens ein Verankerungselement, das zur Verankerung des Implantatkörpers an/in oder zwischen den Wirbelpedikeln oder an Wirbelsäulenversteifungssystemen oder Pedikelschraubensystemen dient und ein Stellelement, das zum Aufspreizen der Schenkel dient, wobei das Stellelement teilweise von dem Verankerungselement umgeben ist.

Hierdurch wird eine zugstabile bzw. eine zug- und druckstabile Verankerung an den hinteren Wirbelsäulenstrukturen oder wahlweise an der hinteren Instrumentationen (Wirbelsäulenversteifungssystem oder Pedikelschrauben), die ihrerseits mit den Wirbelpedikein verbunden sind, erreicht.

Das erfindungsgemäße Implantat rutscht somit trotz Keilform nicht wieder zwischen den Wirbelkörpern heraus sondern ist vielmehr stabil in der vorderen Wirbelsäule zu verankern, so dass es gegen Dislokation gesichert ist.

Zur Verankerung kann das Verankerungselement ein Außengewinde, Dorne oder andere Verankerungsmittel aufweisen, so dass es zwischen zwei Pedikel eingeschraubt werden kann. Auch kann das Verankerungselement an seinem in Richtung des Dornfortsatzes der Wirbelsäule weisenden Endbereich einen Kragen aufweisen, der sich gegen die in Richtung des Dornfortsatzes erstreckende Seite der Pedikel abstützt.

Ein Verankerungselement ist ein jedes Element, das wenigstens Zugkräfte, idealerweise Druck- und Zugkräfte des Implantatkörpers in Richtung des Dornfortsatzes aufnimmt.

Prinzipiell kann ein Verankerungselement auch durch ein Seil oder Band, beispielsweise aus textilern Matelial oder aus Titan, bereitgestellt werden, das an den Pedikeln oder an der Instrumentation direkt oder indirekt befestigt wird und das Implantat auf Zug stabilisiert. Eine Stabilisierung auf Druck ermöglicht diese einfachere Variante nicht.

Das Wirbelimplantat weist weiterhin ein Stellelement auf, das zum Aufspreizen der Schenkel des Implantatkörpers dient und den Implantatkörper mit dem Stellelement verbindet.

Das erfindungsgemäße Implantat kann zunächst als Aufspreizinstrument verwendet werden und dann nach Abschluss der Korrektur als Stabilisierungsimplantat mit dem Verankerungselement und dem Stellelement im Körper verbleiben. Sofern als Stellelement ein einfacher Gewindestab verwendet wird, kann der über das Verankerungselement hinausreichende Bereich des Stellelements nach der Implantation abgezwickt und das Ende des Verankerungselements mit einer Mutter verschlossen werden.

Das Implantat begünstigt durch seine Geometrie und Durchbrechungen bzw. Öffnungen die knöcherne Einheilung.

Bei Verwendung des erfindungsgemäßen Implantats besteht zudem die Möglichkeit, percutan (= minimal invasiv) weitere Korrekturen der Stellung der Wirbelsäule vorzunehmen.

Auch kann die Korrektur in weiteren Sitzungen (nach Dehnung der Weichteile) in lokaler Betäubung mehrfach verstärkt werden.

Das erfindungsgemäße Implantat kann mit anderen Implantaten der (vorderen und hinteren) Wirbelsäulenrekonstruktion kombiniert werden.

Das vorgestellte Implantat eröffnet eine neue Option der Krafteinleitung für die Korrektur und die dauerhafte Sicherung der erreichten Stellung der Wirbelsäule.

Das erfindungsgemäße Implantat kann aus unterschiedlichen Materialien wie Titan, Titanlegierungen, Tantal, Nitinol, Kunststoff wie Polyetheretherketon (PEEK) oder anderen Materialien, die als Implantatwerkstoffe geeignet sind, hergestellt werden.

Das erfindungsgemäße Implantat wird eingesetzt zur Stellungskorrektur und belastungsstabilen Rekonstruktion von in Fehlstellung stehenden Abschnitten der Brust- oder Lendenwirbelsäule.

Das Implantat ersetzt zuverlässig die Tragfunktion eines Knochenspanes oder eines herkömmlichen Zwischenwirbelimplantats ("Cages"). Durch die Ausgestaltung der Außenflächen des Implantatkörpers zum Nachbarwirbel verankert sich der Platzhalter viel sicherer als ein - auch tricortikaler - Knochenspan.

Durch die zusätzliche Verankerung des Keiles an den hinteren Strukturen der Wirbelsäule, insbesondere mit zwei Verankerungselementen, ist zum einen eine symmetrische Fixation möglich, die genau gegen die Dislokationsrichtung gerichtet ist. Zum anderen kann der Keilwinkel und damit die Korrektur ohne großen Aufwand bei liegendem Implantat nachjustiert werden.

Durch Füllung mit kleinen Knochenspänchen ist langfristig die Einheilung und somit biologische Lösung in Form.einer Verknöcherung des Wirbelabschnitts in der korrigierten Stellung erzielbar.

Bei der Implantation werden zunächst die beiden Verankerungselemente, die vorzugsweise Hülsen oder Pedikelschrauben insbesondere mit Innenbohrung sind, zwischen die Pedikel eingeschraubt. Anschließend wird nach Ausräumen der Bandscheibe in den keilförmigen Defekt seitlich der keilförmige Implantatkörper eingeschoben, die Schenkel werden gespreizt und zwischen ihnen das Spreizelement eingeschoben. Nun werden die Stellelemente durch die beiden Verankerungselemente und zwischen den Schenkeln des Implantatkörpers durchgeschoben und dann die Gewinde am Ende der Stellelemente in die beiden Gewinde im Spreizelement eingeschraubt. Jetzt kann durch eine weitere Drehbewegung der Stellelemente das Spreizelement in Richtung des rückwärtigen Bereichs der Wirbelsäule, d.h. in Richtung des Dornfortsatzes, bewegt werden, da das Ende des Stellelements fest mit dem Spreizelement verschraubt und der Implantatkörper zwischen den beiden Wirbelkörpern fixiert ist. Hierdurch werden die Schenkel des Implantatkörpers weiter aufgespreizt. Die entgegen dem Dornfortsatz gerichteten Enden der Verankerungselemente können sich an dem Implantatkörper abstützen.

Verankerungselemente in Form von Hülsen oder Pedikelschrauben mit Innenbohrung dienen neben der Verankerung auch dazu, das Stellelement gegen Überlastung zu schützen. Weiterhin wirken sie als Begrenzung beim Einschrauben des Stellelements und verhindern somit, dass das Implantat in den Spinalkanal gelangt.

Bei sachgemäßem Vorgehen birgt das Implantat keine Risiken. Würde das erfindungsgemäße Implantat zu nah an den Spinalkanal eingebracht oder sich sekundär dorthin verschieben, wäre das Rückenmark gefährdet.

Dadurch, dass bei dem erfindungsgemäßen Implantat im Gegensatz zum Einbringvorgang eines Knochenblocks eine wesentlich bessere Manövriermöglichkeit besteht, ist dieses Risiko kleiner als bei der etablierten Routineversorgung. Durch die neue, konstruktiv bedingte Sicherung durch Fixierung des Implantats an den Wirbelpedikeln über ein Verankerungselement wie einer Schraube oder einer Gewindehülse oder einem Kragen ist das Risiko einer sekundären Verlagerung in das Körperinnere gebannt.

Die Erfindung wird nachfolgend anhand von Ausführungsbeispielen näher beschrieben.

Es zeigen:
- Fig. 1: eine perspektivische Ansicht des erfindungsgemäßen Implantats,

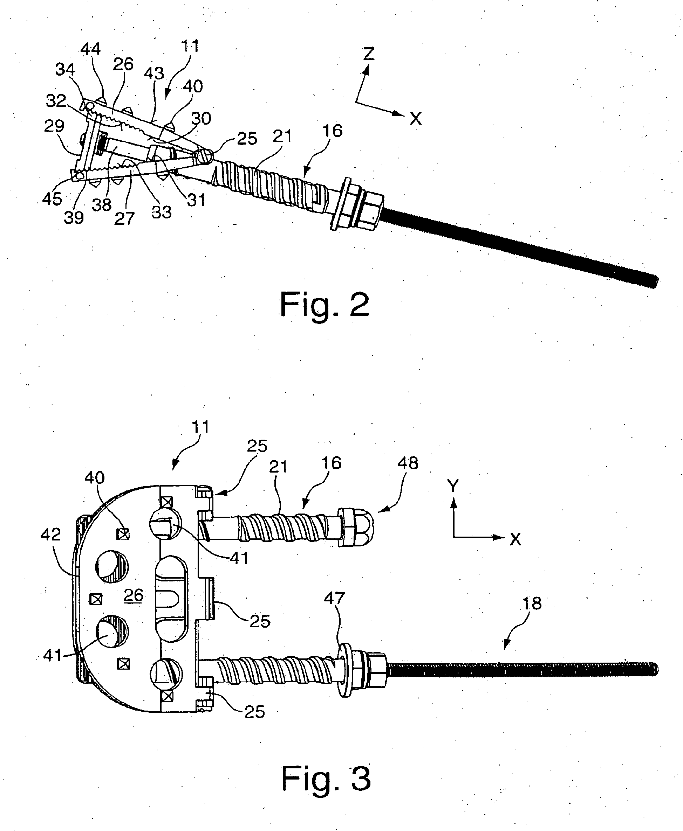

- Fig. 2: die Seitenansicht des Implantats aus Fig. 1,
- Fig. 3: die Draufsicht auf das Implantat aus Fig. 1,
- Fig. 4: die Vorderansicht des Implantats aus Fig. 1,
- Fig. 5: eine perspektivische Ansicht auf den rückwärtigen Bereich des Implantats aus Fig. 1,
- Fig. 6: eine Seitenansicht des erfindungsgemäßen Implantats schräg von oben zwischen zwei Wirbelkörpern/Wirbelpedikeln,
- Fig. 7: eine perspektivische Ansicht des Implantats zwischen zwei Wirbelkörpern/Wirbelpedikeln schräg von oben,
- Fig. 8: eine perspektivische Ansicht des Implantats von hinten (Dornfortsatz) zwischen zwei Wirbelkörpern/Wirbelpedikeln und
- Fig. 9: eine Draufsicht auf das Implantat zwischen zwei Wirbelkörpern/Wirbelpedikein.

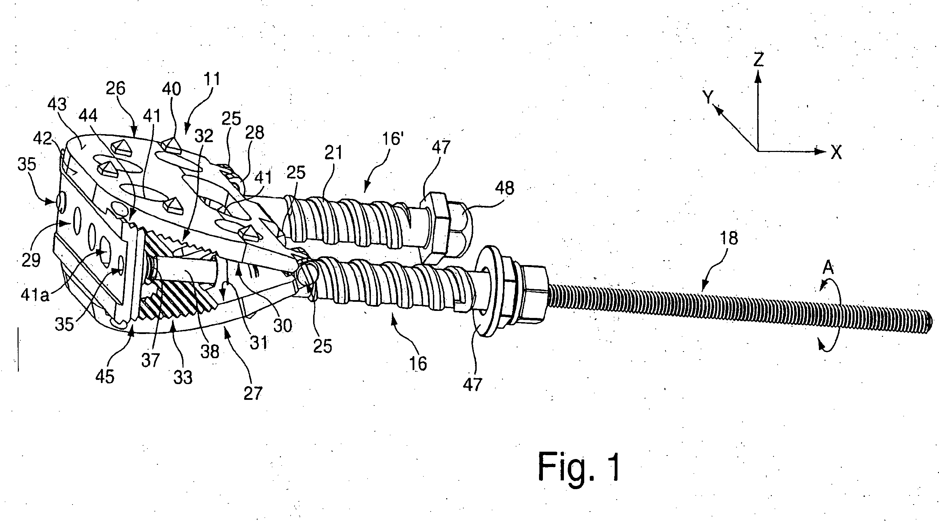

Figur 1 zeigt ein Wirbelsäulenimplantat mit einem Implantatkörper 11, der zwei Implantatschenkel 26, 27 umfasst. Weiterhin umfasst das Wirbelsäulenimplantat zwei hülsenförmige Verankerungselemente 16.

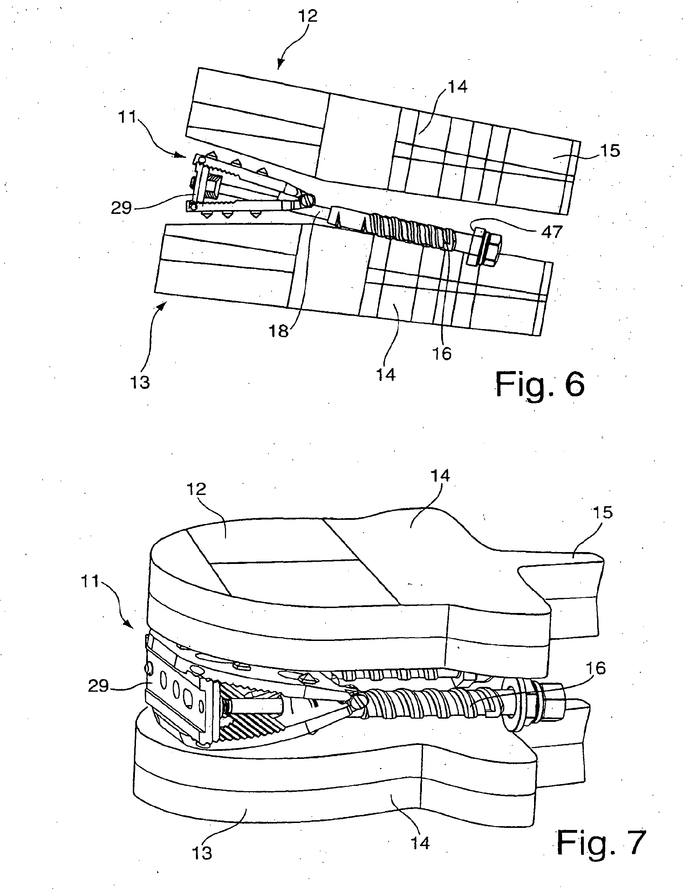

Das vordere Verankerungselement 16 ist mit einem stabförmigen Steilelement (Gewindestab) 18 dargestellt. Bei einer Implantation werden zwei solcher Verankerungselemente 16 mit zwei Stellelementen 18 verwendet, wie in den Figuren 6 ff. dargestellt ist.

Das hintere Verankerungselement 16' in Fig. 1 zeigt das Verankerungselement 16 nach einer Implantation, wenn der überstehende Gewindestab 18 abgetrennt ist (sofern als Stellelement 18 ein einfacher langer Gewindestab verwendet wurde) und auf die dornfortsatzseitige Öffnung des Verankerungselements 16', das an dieser Seite einen Kragen 47 aufweist, eine Mutter 48 aufgeschraubt ist.

Nach der Implantation stützt sich die entgegen dem Dornfortsatz gerichtete Seite des Kragens 47 an der Außenseite des Wirbelpedikels ab.

Die Implantatschenkel 26, 27 des Implantatkörpers 11 sind plattenförmig flach und in der Art eines Halbkreises geformt. An ihrer geraden Kante 28 sind die beiden Implantatschenkel 26, 27 mittels Gelenkelementen 25 miteinander verbunden. An den Außenseiten 43 weisen die beiden Schenkel 26, 27 des Implantatkörpers 11 Dorne 40 auf, die der Verankerung an/in den Wirbelkörpern dienen. Zur Verbesserung der Durchknöcherung umfassen die Schenkel 26, 27 des Implantatkörpers 11 mehrere Öffnungen 41.

An den Innenseiten 30, 31 der Schenkel 26, 27 sind Verzahnungen 32, 33 vorgesehen, die parallel zu der geraden Kante 28 der Schenkel 26, 27 verlaufen und sich etwa von der Mitte des Schenkels in Richtung der halbkreisförmigen Außenkante 42 erstrecken.

Zwischen den beiden Schenkeln 26, 27 ist ein Spreizelement 29 vorgesehen, das an seiner nach oben 34 und unten 39 gerichteten Seite ebenfalls Verzahnungen 44, 45 aufweist. Durch die ineinandergreifenden Verzahnungen 32, 44 und 33, 45 der Schenkel 26, 27 und des Spreizelements 29 und die Ausrichtung der Zähne wird ein selbsthaltender Sitz des Spreizelements 29 zwischen den Schenkeln 26, 27 erreicht.

Auch das Spreizelement 29 weist Öffnungen 41a auf, die - wie die Öffnungen 41 in den Schenkeln 26, 27 - der besseren Durchknöcherung des Implantats dienen.

Zwei Bohrungen 35 mit Innengewinde sind nahe den Längsseiten des Spreizelements 29 vorgesehen. In diese Bohrungen 35 werden die Gewinde 37 am Stabende 38 des Stellelements 18 eingeschraubt, so dass eine starre Verbindung zwischen Stellelement 18 und Spreizelement 29 erzielt wird. Durch Drehen der Stellelemente 18 in Richtung des Pfeils A kann somit das Spreizelement 29 in x-Richtung verschoben werden, wobei das Spreizelement 29 mit seiner Verzahnung 45, 44 in den jeweiligen selbsthaltenden Ruhepositionen der Verzahnungen 32, 33 in den Schenkeln 26, 27 einrastet.

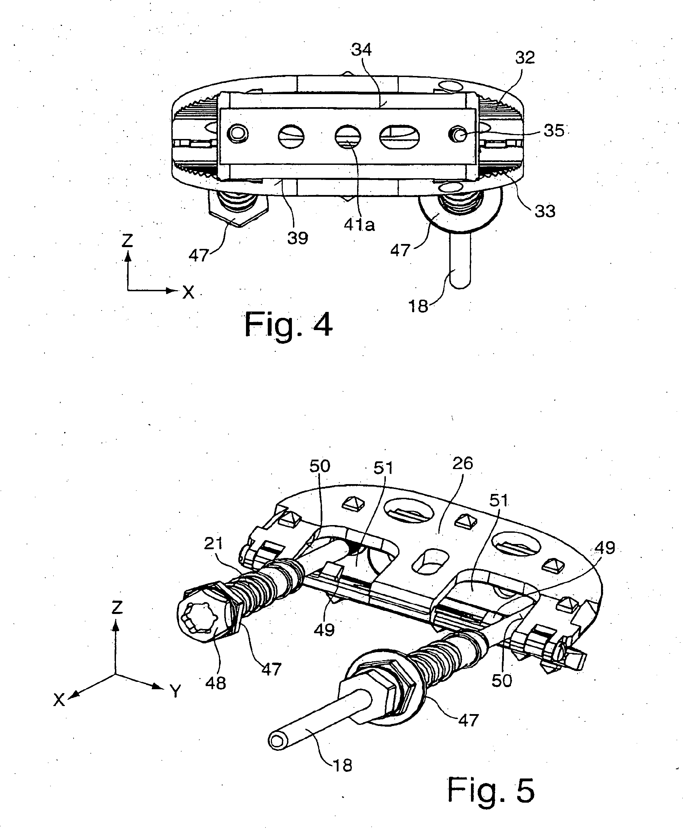

Die Zähne der Verzahnungen 32, 33, 44, 45 sind so orientiert, dass die Verzahnung selbsthaltend ist. Dadurch, dass jeweils zwei Stellelemente 18 und zwei Verankerungselemente 16 vorgesehen sind und die Stellelemente 18 in dem Implantatkörper 11 entlang eines Steges 49 und der Außenkante 50 einer Öffnung 51 in dem oberen Schenkel 26 geführt sind (Fig. 5), wird ein zug- und druckstabiles, in sich stabiles, einfach aufspreizbares Implantat bereitgestellt.

Sofern das nachfolgend beschriebene Verankerungselement 16 starr mit dem Implantatkörper 11 verbunden ist, beispielsweise durch Verschraubung, kann das Innengewinde auch in dem Verankerungselement 16 vorgesehen sein.

Zur Verankerung des Implantatelements 11 sind zwei Verankerungselemente 16 vorgesehen, die vorzugsweise in Form einer Hülse mit einem Schraubgewinde 21 oder einer Pedikelschraube mit Bohrung ausgebildet sind. Bei dieser Ausführungsform wird das Stellelement 18 in der Hülse des Verankerungselements 16 geführt. Hierdurch wird eine verkippfreie Führung des Spreizelements 29 erreicht.

Die Verankerungselemente 16 stützen sich an dem Implantatkörper 11 durch Anlage an der Außenkante 50 der Öffnung 51 und dem Steg 49 ab.

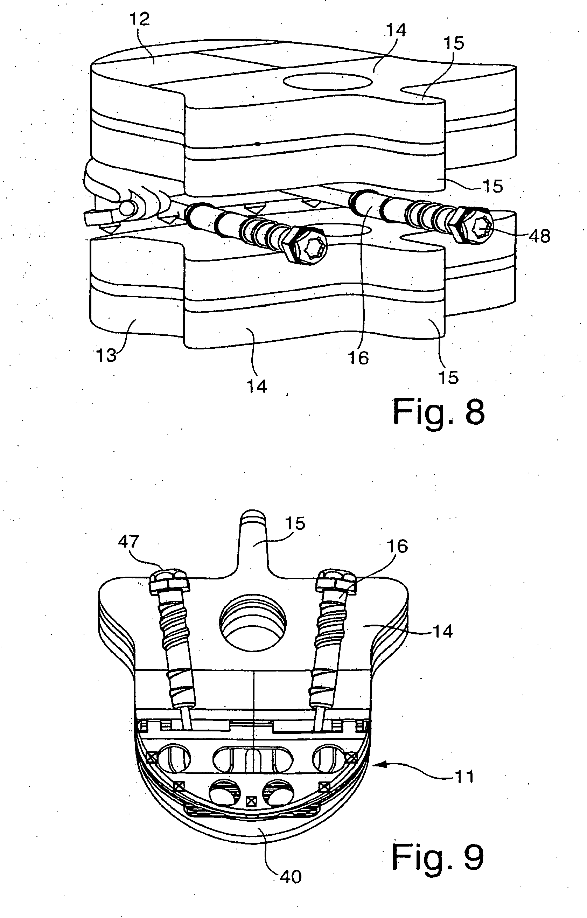

Nach der Implantation ist der Implantatkörper 11 zwischen zwei Wirbelkörpern 12, 13 angeordnet, wie in den Figuren 5 bis 8 näher dargestellt ist. Die aufgespreizten Schenkel bohren sich mit ihren Dornen 40 in die Wirbelkörperaußenfläche ein. Das Verankerungselement 16, das mit dem Implantatkörper 11 direkt oder über das Stellelement 18 verbunden ist und das zwischen zwei Wirbelpedikeln 14 eingeschraubt ist, durchquert den gesamten Wirbelkörper mit Pedikeln und bildet somit eine effektive Stabilisierung und Verankerung des Implantatkörpers gegen ein Herausrutschen aus dem keilförmigen Defekt.

Das Verankerungselement 16 kann allein durch ein entsprechend geformtes schneidendes oder nicht schneidendes Außengewinde, aber auch durch nach außen weisende Dorne oder andere eine Fixierung an den Pedikeln ermöglichende Mittel, bereit gestellt werden. Eine Fixierung an den Pedikeln kann sowohl an den beiden einander gegenüberliegenden Ober- und Unterseiten zweier benachbarter Pedikel erreicht werden, als auch durch Fixierung an der in rückwärtiger Richtung (in Richtung des Dornfortsatzes) weisenden Seite der Pedikel. Somit ist auch eine Verankerung durch entsprechend geformte, mit dem Stab verbundene Kragen oder Unterlagsscheiben, die auf der in Richtung des Dornfortsatzes gerichteten Außenfläche der Pedikel anliegen, möglich.

Das erfindungsgemäße Implantat ist symmetrisch zur Ebene in xz-Richtung.

In den Figuren 5 bis 8 ist das erfindungsgemäße Implantat implantiert zwischen zwei Wirbelkörpern 12, 13 dargestellt. Der Implantatkörper 11 befindet sich zwischen den beiden Wirbelkörpern 12, 13 und die beiden Verankerungselemente 16 sind zwischen den beiden Wirbelpedikeln 14 angeordnet (eingeschraubt) und erstrecken sich mit ihrem einen Ende bis an das Ende der Wirbelpedikel 14. Der Kragen 47 des Verankerungselements 16 stützt sich an der in Richtung des Dornfortsatzes 15 weisenden Außenseite der Wirbelpedikel 14 ab.

## Patentansprüche

1. Wirbelsäulenimplantat, insbesondere für die Brust- und Lendenwirbelsäule, das einen Implantatkörper (11) mit zwei Schenkeln (26, 27) umfasst und zwischen den Schenkeln (26, 27) ein Spreizelement (29) vorgesehen ist, wobei der Implantatkörper (11) zwischen zwei Wirbelkörpern (12, 13) einbringbar ist, das Wirbelsäulenimplantat mindestens ein Element (16) zur Verankerung an/in oder zwischen den Wirbelpedikeln (14) oder dem Wirbelsäulenversteifungssystem oder dem Pedikelschraubensystem umfasst und wobei ein Stellelement (18) vorgesehen ist, das zum Aufspreizen der Schenkel (26, 27) dient und wobei das Verankerungselement (16) eine Schraube, eine Hülse, ein Band oder ein Seil ist und mit dem Implantatkörper (11) verbindbar ist, **dadurch gekennzeichnet, dass** der Implantatkörper (11) keilförmig ist und das Stellelement (18) teilweise von dem Verankerungselement (16) umgeben ist.

2. Wirbelsäulenimplantat nach Anspruch 1, **dadurch gekennzeichnet, dass** das Verankerungselement (16) ein Gewinde (21) und/oder einen Kragen (47) aufweist und vorzugsweise eine Hülse oder eine Pedikelschraube ist.

3. Wirbelsäulenimplantat nach einem der vorangegangenen Ansprüche, **dadurch gekennzeichnet, dass** das Stellelement (18) ein Stab, vorzugsweise ein Gewindestab, ist.

4. Wirbelsäulenimplantat nach einem der vorangegangenen Ansprüche, **dadurch gekennzeichnet, dass** sich das Verankerungselement (16) an dem Implantatkörper (11) abstützt.

5. Wirbelsäulenimplantat nach einem der vorangegangenen Ansprüche, **dadurch gekennzeichnet, dass** die beiden Schenkel (26, 27) halbkreisförmige flache Schenkel sind, die an ihrer geraden Seitenkante (28) schwenkbar miteinander verbunden sind.

6. Wirbelsäulenimplantat nach Anspruch 5, **dadurch gekennzeichnet, dass** zwischen den beiden Schenkeln (26, 27) des Implantatkörpers (11) das Stellelement (18) hindurchführbar ist.

7. Wirbelsäulenimplantat nach Anspruch 5 oder 6, **dadurch gekennzeichnet, dass** an den Innenseiten (30, 31) der Schenkel (26, 27) Verzahnungen (32, 33) vorgesehen sind, das Spreizelement (29) korrespondierende Verzahnungen (44, 45) aufweist und die Verzahnungen (32, 33, 44, 45) in Vorschubrichtung des Stellelements (18) eine Reihe von Ruhepositionen bilden.

8. Wirbelsäulenimplantat nach Anspruch 7, **dadurch gekennzeichnet, dass** die Ruhepositionen selbsthaltend sind.

9. Wirbelsäulenimplantat nach einem der Ansprüche 6 bis 8, **dadurch gekennzeichnet, dass** die Implantatschenkel (26, 27) und/oder das Spreizement (29) Öffnungen (41, 41a) aufweisen, die der besseren Durchknöcherung dienen.

10. Wirbelsäulenimplantat nach einem der Ansprüche 6 bis 9, **dadurch gekennzeichnet, dass** die Implantatschenkel (26,27) Dorne (40) zur Verankerung in den Wirbelkörpern umfassen.

11. Wirbelsäulenimplantat nach einem der vorangegangenen Ansprüche, **dadurch gekennzeichnet, dass** zwei Verankerungselemente (16) vorgesehen sind, die zwischen die Pedikel (14) einschraubbar sind.

12. Wirbelsäulenimplantat nach einem der vorangegangenen Ansprüche, **dadurch gekennzeichnet, dass** zwei Stellelemente (18) vorgesehen sind, die durch die beiden Verankerungselemente (16) durchschiebbar sind.

## Claims

1. A spinal implant, in particular for the thoracic and lumbar spine, comprising an implant body (11) having two branches (26, 27) between which an expansion element (29) is provided, the implant body (11) being insertable between two vertebral bodies (12, 13), the spinal implant comprising at least one element (16) permitting anchoring on/in or between the vertebral pedicles (14) or the spinal fusion system or the pedicle screw system, and an adjusting element (18) being provided which is operable to spread the branches (26, 27) apart, and the anchoring element (16) being a screw, a sleeve, a strap or a cord connectable to the implant body (11), **characterised in that** said implant body (11) is wedge-shaped and said adjusting element (18) is at least partly surrounded by said anchoring element (16).

2. The spinal implant as claimed in claim 1, **characterised in that** the anchoring element (16) has a thread (21) and/or a collar (47) and is preferably a sleeve or a pedicle screw.

3. The spinal implant as claimed in any of the preceding claims, **characterised in that** the adjusting element (18) is a rod, preferably a threaded rod.

4. The spinal implant as claimed in any of the preceding claims, **characterised in that** the anchoring element (16) is supported on the implant body (11).

5. The spinal implant as claimed in any of the preceding claims, **characterised in that** the two branches (26, 27) are semicircular, flat branches which are pivotably connected to each other on their respective straight lateral edges (28).

6. The spinal implant as claimed in claim 5, **characterised in that** the adjusting element (18) may be passed through between the two branches (26, 27) of the implant body (11).

7. The spinal implant as claimed in claim 5 or 6, **characterised in that** toothings (32, 33) are provided on the interior faces (30, 31) of the branches (26, 27), the expansion element (29) is provided with corresponding toothings (44, 45), and the toothings (32, 33, 44, 45) form a series of resting positions in the direction of travel of the adjusting element (18).

8. The spinal implant as claimed in claim 7, **characterised in that** said resting positions are self-locking.

9. The spinal implant as claimed in any one of claims 6 to 8, **characterised in that** the implant branches (26, 27) and/or the expansion element (29) are provided with openings (41, 41a) designed to encourage bone ingrowth into the implant.

10. The spinal implant as claimed in any one of claims 6 to 9, **characterised in that** the implant branches (26, 27) comprise spikes (40) permitting their achoring in the vertebral bodies.

11. The spinal implant as claimed in any of the preceding claims, **characterised in that** two anchoring elements (16) are provided which may be screwed in between the pedicles (14).

12. The spinal implant as claimed in any of the preceding claims, **characterised in that** two adjusting elements (18) are provided which may be slid through the two anchoring elements (16).

## Revendications

1. Implant rachidien, en particulier pour les rachis dorsal et lombaire, lequel comprend un corps d'implant (11) avec deux branches (26, 27) et un élément d'écartement (29) prévu entre lesdites branches (26, 27), le corps d'implant (11) pouvant être placé entre deux corps de vertèbre (12, 13), l'implant rachidien comprenant au moins un élément (16) pour l'ancrage sur/dans ou entre les pédicules vertébraux (14) ou le système de raidissement de la colonne vertébrale ou le système de vis pédiculaires, et un élément de réglage (18) étant prévu, lequel sert à écarter les branches (26, 27), et l'élément d'ancrage (16) étant une vis, un manchon, un ruban ou un câble et étant reliable au corps d'implant (11), **caractérisé en ce que** le corps d'implant (11) est en forme de coin et que l'élément de réglage (18) est partiellement entouré par l'élément d'ancrage (16).

2. Implant rachidien selon la revendication 1, **caractérisé en ce que** l'élément d'ancrage (16) présente un filetage (21) et/ou une collerette (47) et est, de préférence, un manchon ou une vis pédiculaire.

3. Implant rachidien selon l'une quelconque des revendications précédentes, **caractérisé en ce que** l'élément de réglage (18) est une tige, de préférence une tige filetée.

4. Implant rachidien selon l'une quelconque des revendications précédentes, **caractérisé en ce que** l'élément d'ancrage (16) s'appuie sur le corps d'implant (11).

5. Implant rachidien selon l'une quelconque des revendications précédentes, **caractérisé en ce que** les deux branches (26, 27) sont des branches plates semi-circulaires lesquelles sont reliées l'une à l'autre de manière pivotante au niveau de leur bord latéral rectiligne (28) respectif.

6. Implant rachidien selon la revendication 5, **caractérisé en ce que** l'élément de réglage (18) peut être introduit entre les deux branches (26, 27) du corps d'implant (11).

7. Implant rachidien selon la revendication 5 ou 6, **caractérisé en ce que** des dentures (32, 33) sont prévues sur les faces intérieures (30, 31) des branches (26, 27), que l'élément d'écartement (29) présente des dentures correspondantes (44, 45) et que les dentures (32, 33, 44, 45) forment, dans la direction de déplacement de l'élément de réglage(18), une suite de positions de repos.

8. Implant rachidien selon la revendication 7, **caractérisé en ce que** les positions de repos sont automaintenues.

9. Implant rachidien selon l'une quelconque des revendications 6 à 8, **caractérisé en ce que** les branches d'implant (26, 27) et/ou l'élément d'écartement (29) présentent des ouvertures (41, 41a) qui servent à faciliter le passage du tissu osseux pendant sa croissance.

10. Implant rachidien selon l'une quelconque des revendications 6 à 9, **caractérisé en ce que** les branches d'implant (26, 27) comprennent des pointes (40) pour l'ancrage dans les corps de vertèbre.

11. Implant rachidien selon l'une quelconque des revendications précédentes, **caractérisé en ce qu'**il est prévu deux éléments d'ancrage (16) qui peuvent être vissés entre les pédicules (14).

12. Implant rachidien selon l'une quelconque des revendications précédentes, **caractérisé en ce qu'**il est prévu deux éléments de réglage (18) qui peuvent être inséré, de part en part, dans les deux éléments d'ancrage (16).
